# EUROPÄISCHE PATENTSCHRIFT

(11) **EP 1 904 082 B1**
(45) Veröffentlichungstag und Bekanntmachung des Hinweises auf die Patenterteilung: **19.05.2010**
(21) Anmeldenummer: 06795383.6
(22) Anmeldetag: 21.07.2006
(51) Int. Cl.: A61K 38/00, G01N 33/68, G01N 33/92, A61P 3/10

(54) **VERFAHREN ZUR BESTIMMUNG DER KONZENTRATION DER ADIPOZYTAREN FORM DES FATTY ACID BINDUNG PROTEIN (A-FABP, FABP4, aP2)**
METHOD FOR DETERMINING THE CONCENTRATION OF THE ADIPOCYTIC FORM OF THE FATTY ACID BINDING PROTEIN (A-FABP, FABP4, aP2)
PROCÉDÉ POUR DÉTERMINER LA CONCENTRATION D'UNE FORME ADIPOCYTAIRE DE LA PROTÉINE DE LIAISON AUX ACIDES GRAS (A-FABP, FABP4, aP2)

(30) Priorität: 21.07.2005 DE 102005034788
(43) Veröffentlichungstag der Anmeldung: 02.04.2008
(73) Patentinhaber: BioVendor Laboratory Medicine, Inc., 612 00 Brno (CZ)
(72) Erfinder: RUZICKA, Viktor, 616 00 Brno (CZ)
(74) Vertreter: Baumbach, Friedrich
(86) Internationale Anmeldenummer: PCT/IB2006/002383
(87) Internationale Veröffentlichungsnummer: WO 2007/063363

(56) Entgegenhaltungen:
- WO-A-00/66784
- XU AIMIN ET AL: "Adipocyte fatty acid-binding protein is a plasma biomarker closely associated with obesity and metabolic syndrome." CLINICAL CHEMISTRY MAR 2006, Bd. 52, Nr. 3, März 2006 (2006-03), Seiten 405-413, XP002435912 ISSN: 0009-9147
- DATABASE CA [Online] CHEMICAL ABSTRACTS SERVICE, COLUMBUS, OHIO, US; STEJSKAL, DAVID ET AL: "Assessment of clinical relevance of serum adipocyte fatty acid binding protein concentration determined by new ELISA method in patients with diagnosed metabolic syndrome" XP002436246 gefunden im STN Database accession no. 146:77391
- FISHER R M ET AL: "Fatty acid binding protein expression in different adipose tissue depots from lean and obese individuals" DIABETOLOGIA, Bd. 44, Nr. 10, Oktober 2001 (2001-10), Seiten 1268-1273, XP002435913 ISSN: 0012-186X
- BIOVENDOR: "ELISA Kits & Other Assays" INTERNET ARTICLE, [Online] 5. Juni 2005 (2005-06-05), XP002436329 Gefunden im Internet: URL:http://web.archive.org/web/20050605002 411/http://www.biovendor.com/> [gefunden am 2007-06-05]
- BIOVENDOR: "Antibody, Immunoaffinity Purified" DATA SHEET- RD181037050, [Online] 5. Juni 2005 (2005-06-05), XP002436346 Gefunden im Internet: URL:http://web.archive.org/web/20050605002 411/http://www.biovendor.com/>
- INTERNET CITATION, [Online] Biovendor recombinant FABP-4 protein product data sheet Gefunden im Internet: <URL:http://web.archive.org/web/20050605002 411/http://www.biovendor.com/> [gefunden am 2004-01-26]

## Beschreibung

Die Erfindung betrifft ein Verfahren zur Bestimmung der Konzentration der adipozytären Form des fatty acid binding protein (A-FABP, FABP4, P2) für die Diagnostik des Metabolischen Syndroms,

Anwendungsgebiete sind die Medizin und hier besonders die medizinische Diagnostik.

Das Metabolische Syndrom, auch als Syndrom X, Insulinresistenz-Syndrom oder Multiples Metabolisches Syndrom bezeichnet, stellt eine Störung der Verstoffwechslung von Lipiden, Kohlenhydraten, Proteinen, Mineralstoffen usw. des Organismus dar, die durch Erbfaktoren und/oder Lebensbedingungen hervorgerufen werden kann. Zum Metabolischem Syndrom gehören: Insulinresistenz, Dyslipidämie, arterielle Hypertonie und Adipositas.

Insulinresistenz wird als eine Unempfindlichkeit gegenüber dem körpereigenen Insulin definiert. An der Insulinresistenz leiden nicht nur die Patienten mit Diabetes Typ II, sondern auch ca. 20% der praktisch gesunden, nicht übergewichtigen Menschen. Die Ursachen der Insulinresistenz sind bis heute noch nicht eindeutig geklärt. Zu den Kennzeichen der Insulinresistenz zählen durch die Hyperinsulinämie und Gluko- und Lipotoxität hervorgerufenen Erschöpfung von β-Zellen, ihre Desensibilisierung und Apoptose.

Dyslipidämie ist eine Störung des Fettstoffwechsels in Form von Hypercholesterinämie, Hypertriglyceridämie, Hyperlipidämie (erhöhte Blutfettwerte als Oberbegriff). Diese ist charakterisiert durch hohe Triglyzerid- und niedrige HDL-Cholesterin-Werte, kleine, dichte LDL-Partikel und einen hohen Gehalt an freien Fettsäuren. Das Spektrum der Betroffenheit reicht von der "gewöhnlichen" (polygenen) Hypercholesterinämie (ca. 10% der Bevölkerung) bis zu seltenen, genetisch bedingten Lipidstoffwechselstörungen. Dyslipidämien sind Risikofaktoren für die Atherosklerose, insbesondere für die koronare Herzkrankheit. Darüber hinaus tragen die freien Fettsäuren zur Aufrechterhaltung des Zustandes der Insulinresistenz bei.

Kardiovaskuläre Erkrankungen und insbesondere die koronare Herzkrankheit (KHK) stellen die prognostisch wesentlichen komplikationen des metabolischen Syndroms und damit auch die Haupttodesursache dieser Patientengruppe dar.

Ziel der Erfindung ist die Früherkennung und/oder die Beobachtung des metabolischen Syndroms. Der Erfindung liegt die Aufgabe zu Grunde, Verfahren zu entwickeln, mit denen es möglich ist, geeignete Parameter zur Risikoabschätzung und Verlaufsbeobachtung bei der Diagnostik und Therapie des metabolischen Syndroms.

Die Erfindung wird gemäß Anspruch 1 und Anspruch 19 realisiert, die Unteransprüche sind Vorzugsvarianten. Der Erfindung liegt der Kerngedanke zugrunde, dass die Konzentration von A-FABP in Serum oder Plasma bestimmt wird und zur Diagnose des metabolischen Syndroms verwendet wird.

Eine ganz zentrale Rolle bei der Entstehung, Entwicklung und Verlauf des metabolischen Syndroms und des Diabetes Typ II spielen die Fettzellen und der Fettstoffwechsel. Ein zytoplasmatisches Protein der Fettzelle ist die adipozytäre Form des fatty acid binding protein (abgekürzt A-FABP, FABP4 oder P2), welches längere Fettsäuren, Prostanoide, Retinsäure und ähnliche nicht wasserlösliche Molekule im Zytoplasma der Fettzelle transportiert. Die Konzentration des A-FABP wurde im Serum von Normalpersonen und Diabetikern Typ II verglichen, wobei der Body Mass Index als Hinweis auf den Anteil des Fettgewebes an der Körpermasse der untersuchten Personen berücksichtigt wurde. Obwohl A-FABP ein intrazelluläres Protein der Fettzellen ist, das in der Zirkulation kaum vorkommen sollte, finden sich in normalen gesunden Personen im Serum A-FABP-Spiegel im Nanogrammbereich (ng/ml).
Da die A-FABP Spiegel im Serum etwa doppelt so hoch liegen wie die des adipozytaeren Hormons Leptin (Leptinkonzentrationen ± 8 ng/ml; A-FABP liegt bei ± 16-20 ng/ml), liegt es nahe, dass A-FABP aktiv in die Zirkulation sezerniert wird. Ferner ist es nicht auszuschliessen, dass A-FABP noch eine andere (Signal-) Wirkung zuzuschreiben ist, die von seiner intrazellulären Funktion unabhängig ist. Vollkommen unerwartet war jedoch die Feststellung, dass die Messung der Konzentration von A-FABP in Körperflüssigkeiten für die Feststellung und Beobachtung des Metabolischen Syndroms, geeignet ist.

Der Erfindung liegt also die vollkommen überraschend erhaltene Erkenntnis zugrunde, dass ein signifikant erhöhter Gehalt von A-FABP in Serum oder Plasma mit dem metabolischen Sydrom und in Verbindung steht.

Die Erfindung betrifft deshalb ein Verfahren, bei dem die Konzentration des F-ABP im Serum (Fettsäuretransportkapazität) zur Diagnose des metabolischen Syndroms genutzt wird. Weiterhin vollkommen unerwartet war die Festellung, dass die Konzentration von A-FABP in Körperflüssigkeiten, insbesondere Serum, signifikant mit der Konzentration von anderen Parametern des metabolischen Syndroms, von Diabetes Typ II, Insulinresistenz, Obesität und verwandten Störungen des Stoffwechsels korreliert. Zur Diagnose und Beobachtung des Metabolischen Syndroms und der genannten Stoffwechselstörungen wird die Konzentration von A-FABP in Körperflüssigkeiten und/oder daraus verdünnten Lösungen bestimmt.
Für die Umsetzung der Erfindung sind immunometrische Systeme, insbesondere in Kombination mit einer optischen und/oder biosensorischen Auswertung besonders geeignet, da sie eine einfache Bestimmung der Konzentration von A-FABP in bzw. aus Körperflüssigkeiten erlauben. Verwendbar sind dabei verschiedenste Systeme, mit deren Hilfe sich die Konzentration von A-FABP bestimmen läßt, beispielsweise durch die parallele oder aufeinanderfolgende Vermessung von verschiedenen, Konzentrationen der zu bestimmenden Körperflüssigkeit, insbesondere wenn daneben eine Normalisierung/Eichung mit definierten bzw. bekannten A-FABP-Konzentrationen durchgeführt wird, durch Titration oder durch die Verwendung verschiedener definierter Konzentration an Fängermolekülen, die an einer Festphase/Trägermaterial gebunden sind und Affinität zu A-FABP oder davon abgeleiteten Strukturen haben.

Günstig sind dabei solche Verfahren, die Trägermaterialien umfassen, an deren Oberfläche Substanzen immobilisiert sind, die zur Bestimmung der Konzentration von A-FABP in bzw. aus Körperflüssigkeiten geeignet sind. Dies können insbesondere Moleküle oder aus diesen zusammengesetzte Stoffe, vorzugsweise Proteine, sein, die Affinität zu F-ABP haben, zum Beispiel anti- F-ABP Antikörper. Möglich ist auch die Verwendung von immobilisierten Substanzen, die Affinität zu Teilfragmenten von A-FABP, insbesondere Peptidketten, oder aus Teilfragmenten zusammengesetzten Peptiden haben.
Als Trägermaterial sind alle möglichen Materialien oder beschichtete Materialien geeignet, an deren Oberfläche sich Moleküle immobilisieren lassen, insbesondere Nitrocellulose bzw. PVDF oder andere Materialien zur biophysikalischen Immobilisierung, z.B. in Vertiefungen von ELISA-Platten. Aber auch eine Immobilisierung über elektrostatische Wechselwirkung oder eine Immobilisierung durch chemische Bindung, beispielsweise bei Verwendung sogenannter (Cross-) Linker, ist möglich. Geeignet zur Immobilisierung, insbesondere für biosensorische Messungen, beispielsweise von Oberflächenplasmonenresonanz oder elektrochemischen Größen, ist etwa auch Gold oder beschichtete Goldoberflächen. Vorteilhaft für die Durchführung des erfindungsgemäßen Verfahrens sind insbesondere Trägermaterialien mit zumindest teilweise planar gebildeten Oberflächen, zum Beispiel Glas- oder Kunstoff-Chips oder ELISA-Platten, da sie eine besonders einfache Umsetzung des erfindungsgemäßen Verfahrens, beispielsweise mit Hilfe von Scannern oder ELISA-Readern, erlauben. Neben planaren Oberflächen, auf denen ortsadressierte Bereiche, zum Beispiel Spots, mit immobilisierten Fängermolekülen, insbesondere mit anti-AFABP-Antikörpem gebildet sind, zum Beipiel auf Oberflächen von Cellulose oder Chips, sind insbesondere auch die Bodenflächen der Vertiefungen von ELISA-Platten zur Bestimmung der A-FABP-Konzentration aus Körperflüssigkeiten geeignet.

Als immunometrisches System bietet sich insbesondere der sogenannte Enzyme Linked Immunosorbent Assay (ELISA) an oder vergleichbare Systeme, die Farbstoffmarkierte Moleküle verwenden, wodurch eine besonders hohe Sensitivität des Systems zur Bestimmung der A-FABP-Konzentration ermöglicht wird. Dabei wird ein Trägermaterial, auf dem eine oder mehrere Substanzen, z.B. Antikörper, mit Affinität zu A-FABP oder abgeleiten Molekülen immobilisiert sind, mit Körperfüssigkeit oder Lösungen, die Körperflüssigkeit enthalten, in Kontakt gebracht, so dass das A-FABP bzw. die abgeleitete Form/Sequenz, das sich gegebenfalls in der Lösung mit Körperflüssigkeit befindet, mit den Substanzen in der Nähe der Oberfläche des Trägermaterials assoziieren kann. Dazu kann die Köperflüssigkeit in einer Inkubationslösung, z.B. Blockierungspuffer, gelöst sein, die beipielsweise durch Verminderung unspezifischer Wechselwirkungen zu einer Verbesserung des Signal-/Rausch-Verhältnisses führt. Zum Nachweis des assoziierten A-FABP oder abgeleiteter Moleküle an der Oberfläche wird, meist nach einem Waschschritt, eine gelöste Substanz, vorzugsweise anti-A-FABP Antikörper, auf das Trägermaterial gebracht, wobei die gelöste Nachweissubstanz ebenfalls Affinität zu A-FABP hat und an das auf dem in der Nähe des Trägermaterials assoziierte A-FABP bindet.
Die Detektion der an A-FABP bzw. davon abgeleiteten Moleküle gebundenen Nachweissubstanz/Antikörper kann auf unterschiedlichste Weise erfolgen.
Möglich ist, daß die Nachweissubstanz bzw. anti-A-FABP-Antiköper bereits mit einer Markierung versehen ist, beispielsweise mit einem Farbstoff, insbesondere Fluoreszenzfarbstoff, oder mit einem Enzym, das ein Substrat, beispielsweise aus einer Detektionslösung, optisch nachweisbar umsetzen kann, z.B. Meerrettich-Peroxidase oder alkalische Phosphatase. Die Nachweissubstanz, insbesondere Antikörper, kann aber auch mit einem Molekül, z.B. Biotin versehen sein, das wiederum Affinität zu einem anderen markierten Stoff, z.B. Streptavidin, hat.
Ist die Nachweissubstanzlanti-A-FABP-Antikörper nicht selbst markiert, sind insbesondere markierte Sekundärantikörper geeignet, die, meist nach einem Waschschritt, zugegeben werden und die Nachweissubstanz/anti-A-FABP-Antikörper an der Oberfläche des Trägermaterials sichtbar machen bzw. die nachfolgende optische Auswertung ermöglichen.
Für die Diagnose und Erforschung des metabolischen Syndroms, seiner Frühformen, Begleit- und Folgeerkrankungen, Diabetes Typ II, Insulinresistenz, Obesität oder verwandten Störungen des Stoffwechsels ist auch vorteilhaft geeignet, daß die erfindungsgemäß gemessenen A-FABP-Konzentration mit anderen Kenngrößen, bspw. dem Alter oder Geschlecht, dem Body-Mass-Index oder mit Komponenten des Stoffwechsels, die für das metabolische Syndrom kennzeichnend sind, insbesondere Insulin (bpsw. Quicki-Werte), Glucose, Triglyceride, Adiponectin, Leptin, Gesamt-Cholesterin, HDL-Cholesterin und/oder LDL-Cholesterin in Bezug gesetzt wird. Durch die Verbindung von A-FABP mit wenigstens einem weiteren Parameter kann vorteilhafterweise auch eine Vorhersage von anderen Parametern des Metabolischen Syndroms erfolgen.
Duch die Umsetzung der Erfindung läßt sich auf einfachste Weise auf Kenngrößen des metabolischen Syndroms zurückschließen, womit eine besonders einfache und kostengünstig umzusetzende Diagnose des metabolischen Syndroms ermöglicht wird. Dadurch ist beispielsweise auch keine Entnahme von Gewebeproben nötig, da sich direkt auf die krankhaften Vorgänge im Gewebe bzw. in den Zellen schließen läßt. Vorteilhaft ist insbesondere auch, dass bereits Frühstadien des Metabolischen Syndroms detektierbar sind. Dadurch wird es auch erstmalig möglich, bereits frühzeitig vor dem Auftreten der mit dem Metabolische Syndrom in Verbindung stehenden Krankheitserscheinungen Gegenmaßnahmen für den Patienten, beispielsweise durch Verordnung einer Diät oder Sport oder durch Verabreichung einer geeigneten Medizin, einzuleiten.

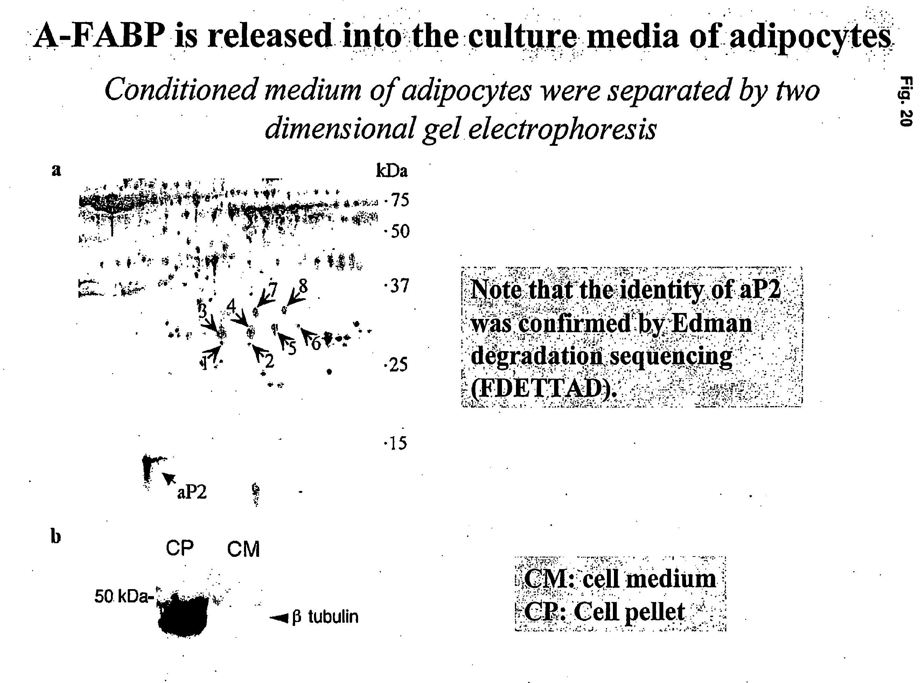

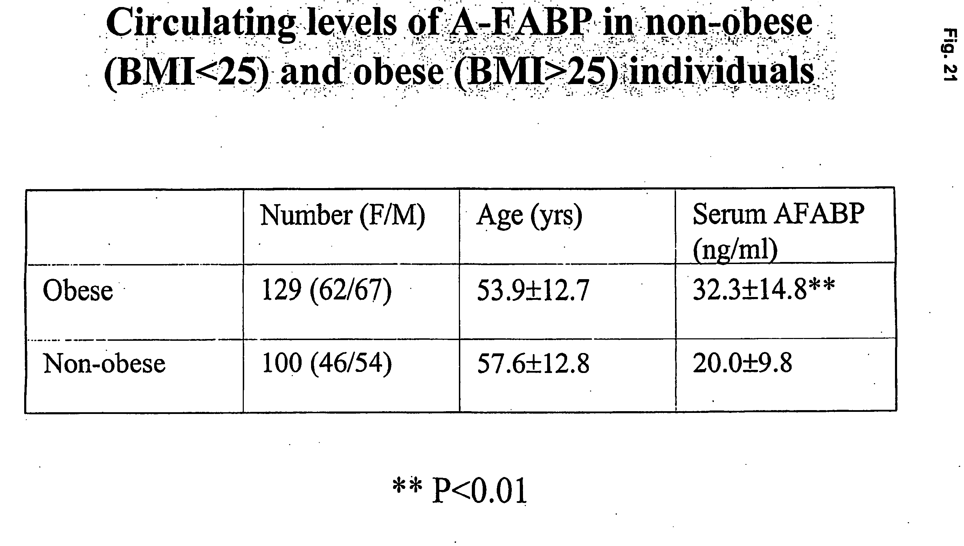

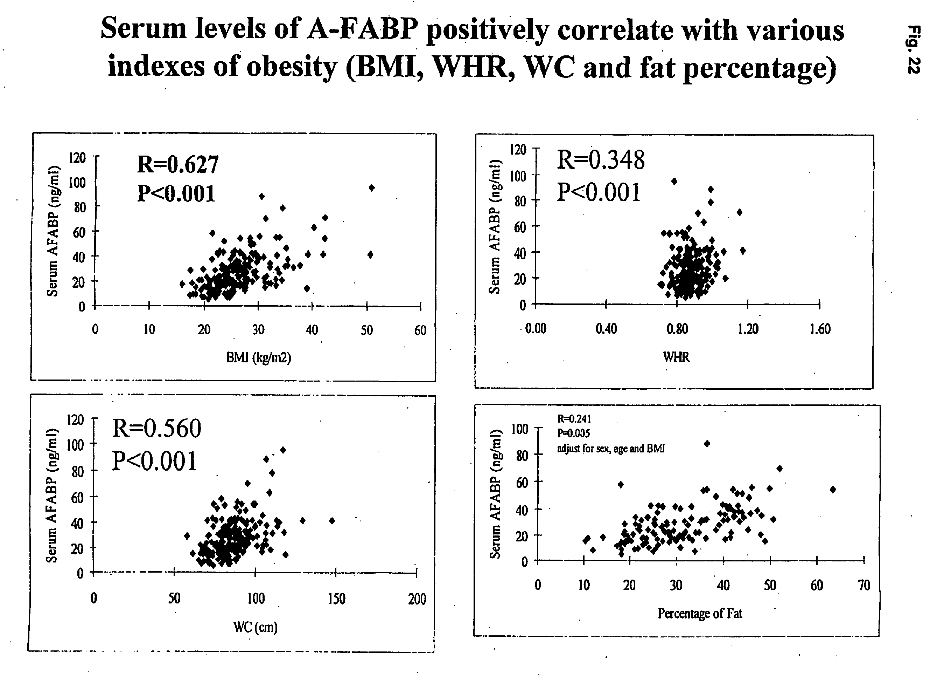

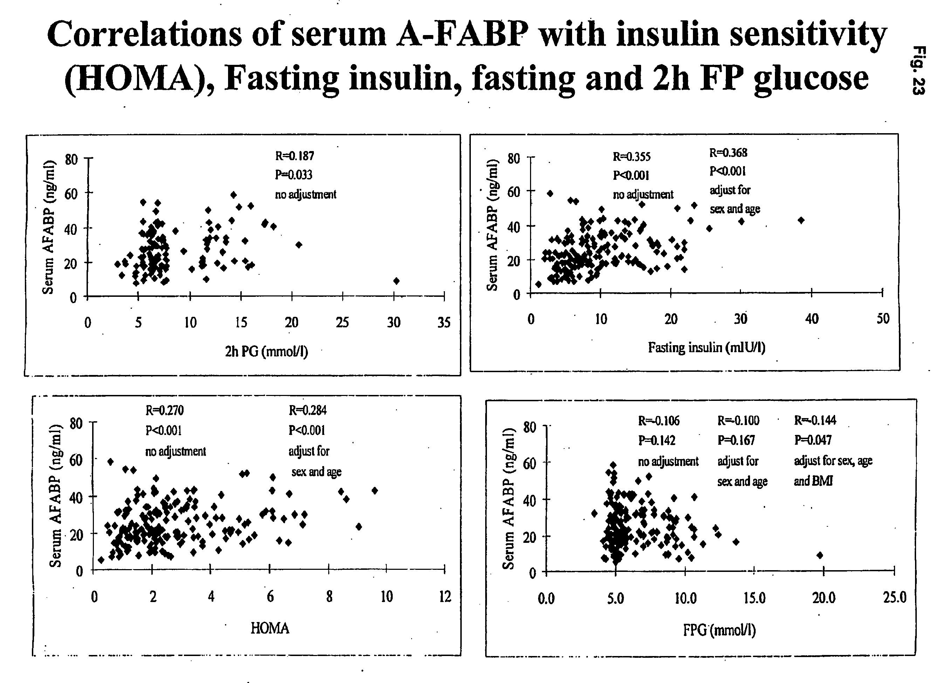

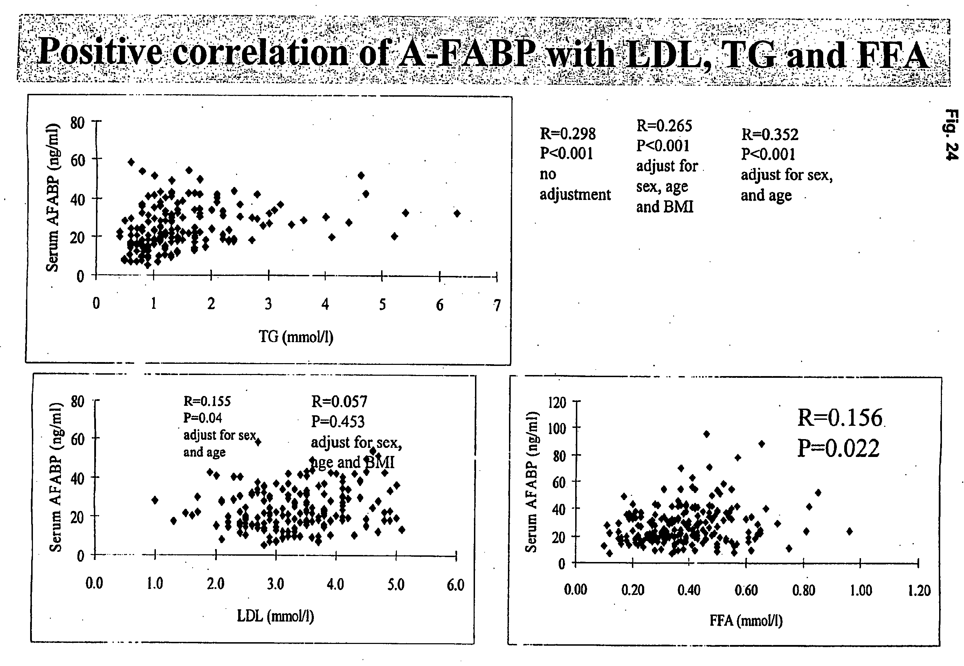

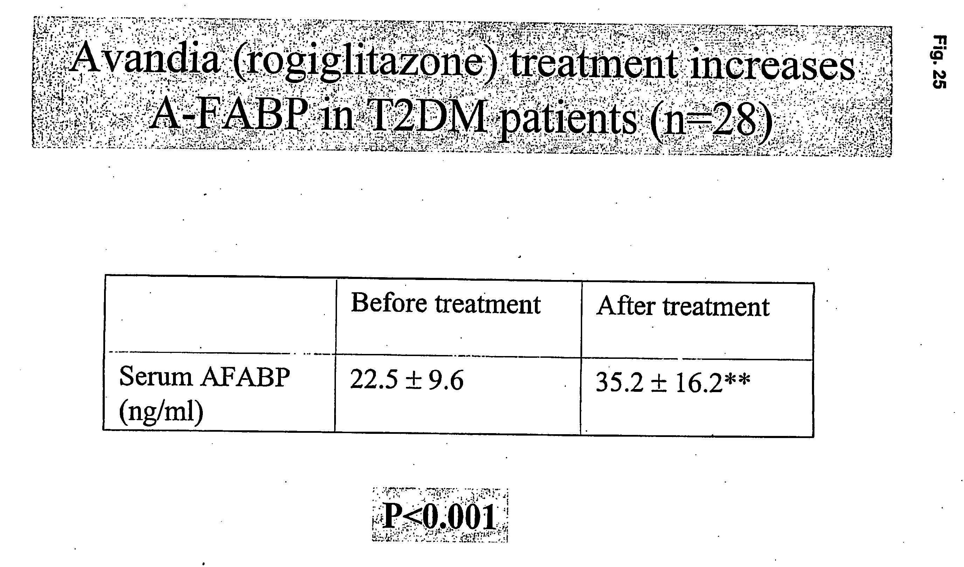

Durch die Erfindung wird auch die Nutzung von A-FABP als hormonähnliche Substanz zur Beeinflussung des Fett-, Zucker- und Energiestoffwechsels ermöglicht (vgl. Figur 20). Wenn eine positive Wirkung von A-FABP vorliegt, ist es oder seine Fragmente oder seine Mimetika als Arzneimittel anwendbar. Bei negativer Wirkung sind A-FABP-Blocker von medizinischem Nutzen.

Figur 20 zeigt eine zweidimensionale Elekrophorese des Zellkulturüberstandes einer adipozytären Kultur, in der ein sehr großer und deutlicher Spot des A-FABP zu sehen ist (die Identität des Proteins wird mittels N-terminaler Sequenzierung mit einem Massenspektrometer bestimmt). Die Tatsache, das kein beta-Tubulin (ein intrazelluläres Strukturprotein) im Überstand mit dem Western Blot nachzuweisen war, deutet darauf hin, dass das A-FABP NICHT durch den Zerfall der Fettzellen, sondern wirklich durch Sekretion in das Medium gelangt.

Die Erfindung liefert somit eine vollkommen neue Möglichkeit der Risikoabschätzung und der Verlaufsbeobachtung bei der Diagnostik und Therapie des Metabolischen Syndroms und des Diabetes Typ II.

Die Erfindung soll nachfolgend durch Anwendungsbeispiele näher erläutert werden:

### Ausführungsbeispiel 1:

Bei 486 Probanden wurde A-FABP im Serum bestimmt. Es handelte sich um 100 normgewichtige gesunde Personen, 100 Patienten mit Dyslipidämie ohne Obesität und ohne andere Merkmale des metabolischen Syndroms, 86 Diabetiker Typ II, 100 oböse und 100 Patienten mit metabolischem Syndrom. Neben A-FABP wurden noch folgende Parameter bestimmt: Body Mass Index (BMI), Glukose, Insulin, quantitativer Insulin-Sensitivitaets-Check-Index (QUICKI), Adiponectin und E-FABP (epidermal fatty acid binding protein):

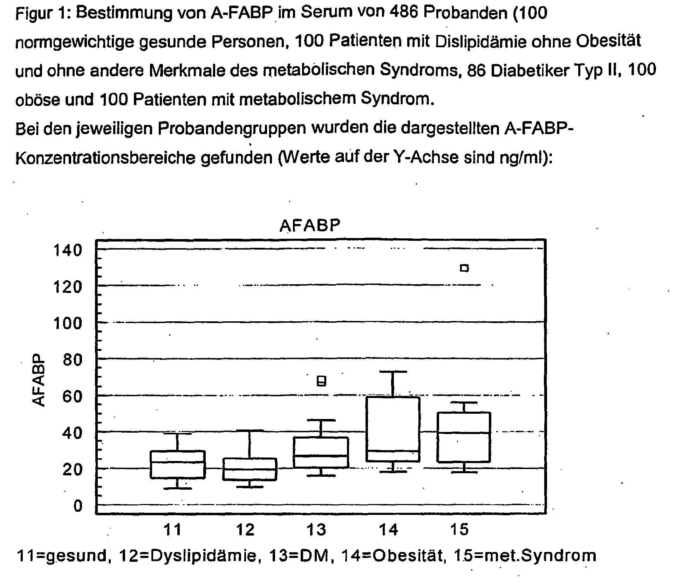

Bei den jeweiligen Probandengruppen wurden A-FABP- Konzentrationsbereiche, wie in Figur 1 dargestellt, gefunden.

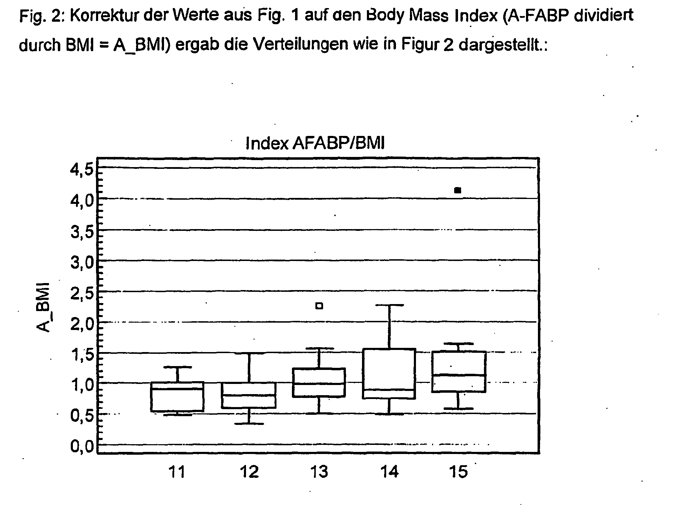

Eine Korrektur auf den Body Mass Index (A-FABP geteilt durch BMI) resultierte in Verteilungen gemäß Figur 2.

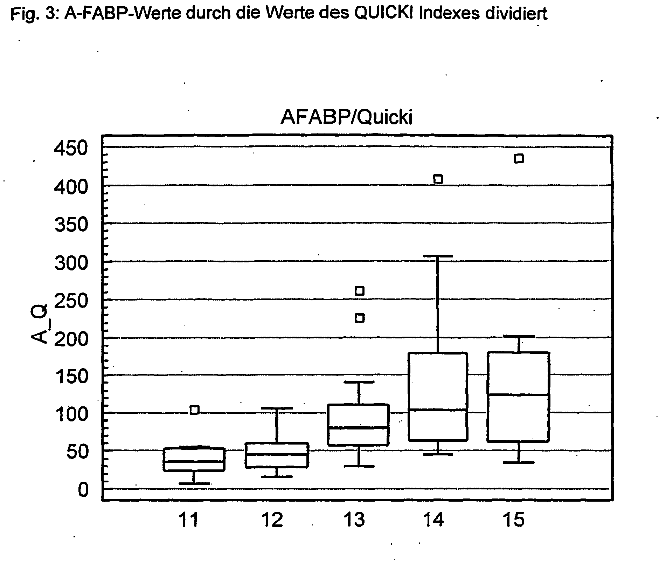

Nach der Teilung der A-FABP-Werte durch die Werte des QUICKI Indexes ergab sich die in Figur 3 dargestellte Verteilung.

Aus den gemessenen Daten war vollkommen überraschend und deutlich ersichtlich, dass die A-FABP-Spiegel sehr wirksam zwischen gesunden Normalpersonen und Patienten mit metabolischem Syndrom diskriminieren, und zwar unabhängig vom Body Mass Index.

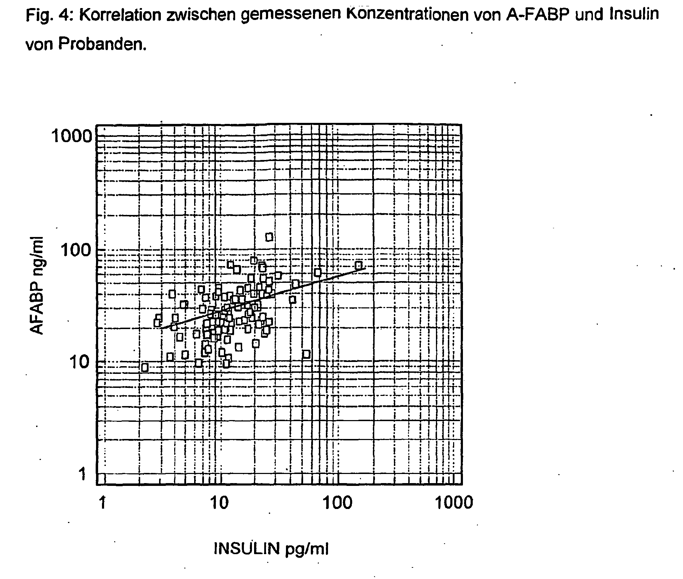

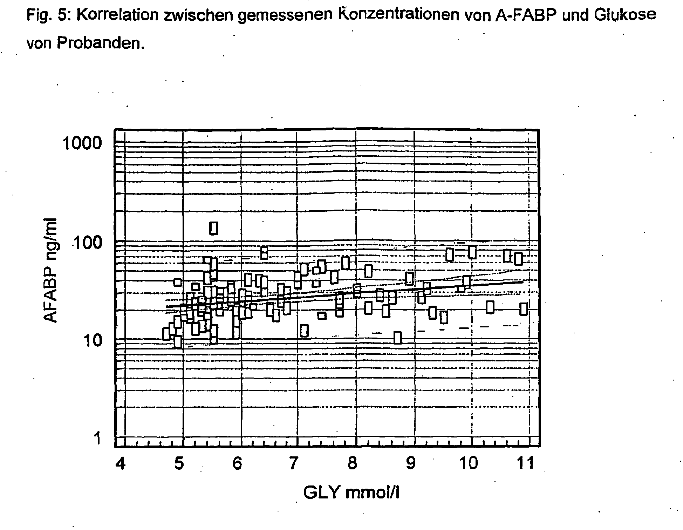

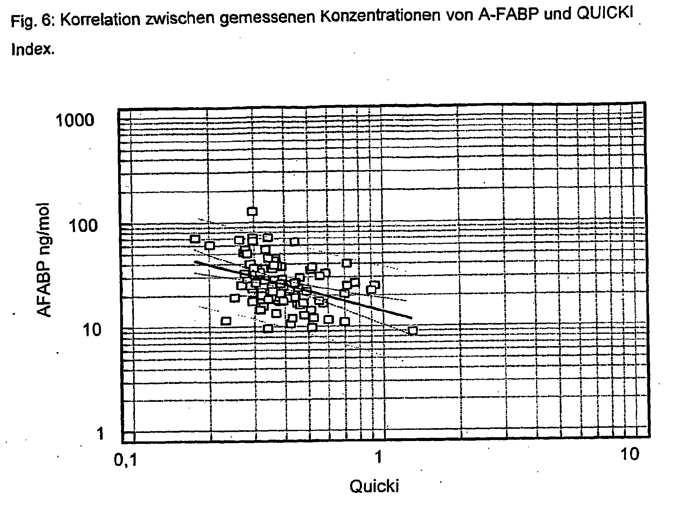

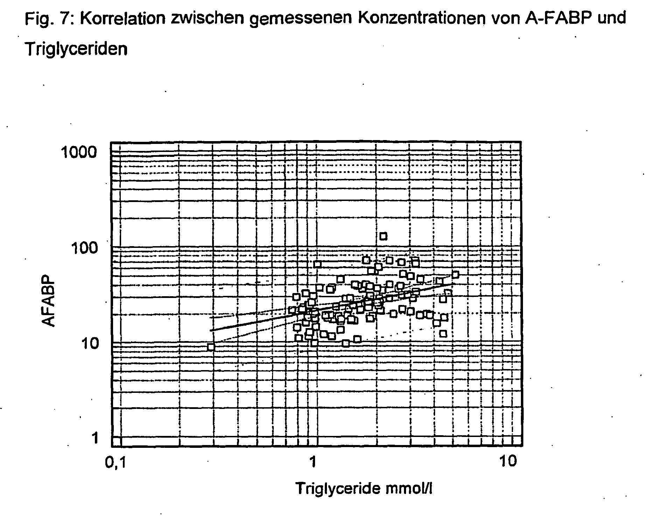

Es wurden weiterhin die Konzentrationen von Glukose, Insulin, quantitativem Insulin-Sensitivitäts-Check-Index (QUICKI) und Triglyceriden gemessen und mit den gemessenen Werten von A-FABP in Korrelation gesetzt (Figuren 4-7).
Es war deutlich erkennbar, daß die gemessenen Werte von Glukose, Insulin, QUICKI und Triglyceriden, also kennzeichnende Parameter für das Metabolische Syndrom, in direkter Korrelation mit der Konzentration des gemessenen A-FABP stehen.
Zieht man dazu noch die Werte aus Figur 1 und Figur 2 heran, wird deutlich, dass die gemessenen Werte des A-FABP aus Körperflüssigkeiten in direktem Zusammenhang mit dem Metabolischen Syndrom und den verwandten Stoffwechselerkrankungen stehen.

### Ausführungsbeispiel 2

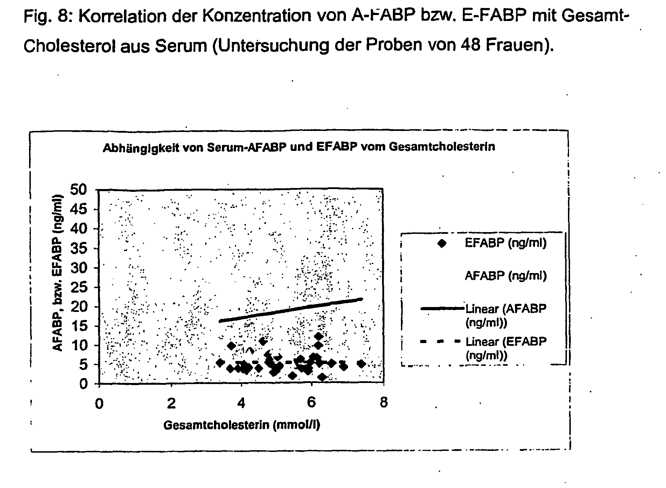

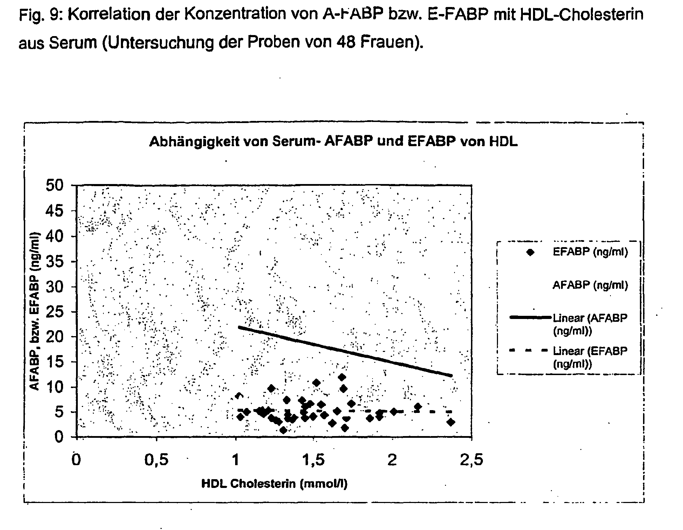

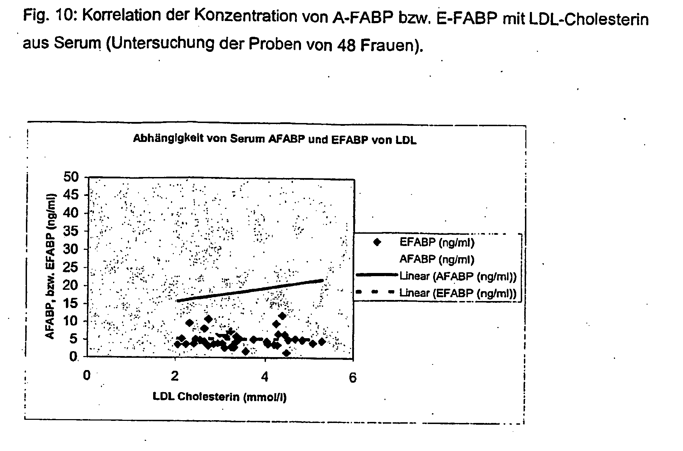

Von 48 Frauen, deren Serumproben zur Verfügung standen unter welchen sich auch Frauen mit metabolischem Syndrom befinden könnten, wurden die Konzentrationen von A-FABP, E-FABP, Gesamt-Cholesterin, HDL-Cholesterin und LDL-Cholesterin bestimmt. Die Auftragung der Werte von A-FABP bzw. E-FABP jeweils gegen die Werte von Gesamt-Cholesterin (Figur 8), HDL-Cholesterin (Figur 9) und LDL-Cholesterin (Figur 10), zeigte deutlich, dass A-FABP eng mit den wichtigsten Parametern des metabolischen Syndroms, von Diabetes Typ II, Insulinresistenz, Obesität (Obesitas/Adipositas/Fettsucht) und verwandten Störungen des Stoffwechsels korreliert.

### Ausführungsbeispiel 3

Herstellung des rekombinanten humanen A-FABP:

cDNA-Sequenz:
ATGTGCGATGCGTTTGTGGGCACCTGGAAACTGGTTAGCAGCGAAAACTTCGATGATTACAT
GAAAGAAGTGGGCGTTGGTTTTGCGACCCGCAAAGTTGCGGGTATGGCGAAACCGAACATGA
TTATCAGCGTGAACGGCGATGTGATTACCATCAAAAGCGAAAGCACCTTCAAAAACACCGAA
ATCAGCTTTATCCTGGGCCAGGAATTTGATGAAGTGACCGCGGATGATCGTAAAGTGAAAAG
CACCATCACCCTGGATGGTGGTGTTCTGGTGCATGTGCAGAAATGGGATGGCAAAAGCACCA

CCATCAAACGCAAACGCGAAGATGATAAACTGGTGGTGGAATGCGTGATGAAAGGTGTTACC
AGCACCCGTGTTTATGAACGTGCG

Aminosäuresequenz:
MCDAFVGTWKLVSSENFDDYMKEVGVGFATRKVAGMAKPNMIISVNGDVITIKSESTFKNTE
ISFILGQEFDEVTADDRKVKSTITLDGGVLVHVQKWDGKSTTIKRKREDDKLVVECVMKGVT
STRVYERA

Das exprimierte Protein enthielt keine zusätzlichen Aminosäuren oder Tags.
**Proteingrösse:** 132 Aminosäuren ; Molare Masse: ca. 14720 Da
**Expressionsstamm:** *E*.*coli*: BL21DE3(pRSET-FABP4)-1
**Expressionsverfahren:**
   - SOB-Mediumo (4,5 L) wurde mit einer Übernachtkultur von *E.coli* BL21DE3(pRSET-FABP4)-1 beimpft
   - Kultivierung verlief bei 37° C auf einem Schüttler.
   - Induktor IPTG wurde nach 3,5 Stunden der Kultivierung bei OD = 0,69 dazugegeben.
   - Die Expression wurde nach 4 Stunden beendet. Die optische Dichte erreichte OD = 1,52.
   - Die Bakterien wurden abzentrifugiert und eingeengt auf eine theoretische OD = 100 in 68,4 ml PBS Puffer.
   - Durch Ultraschallbehandlung der Bakterien wurde lösliches A-FABP gewonnen.

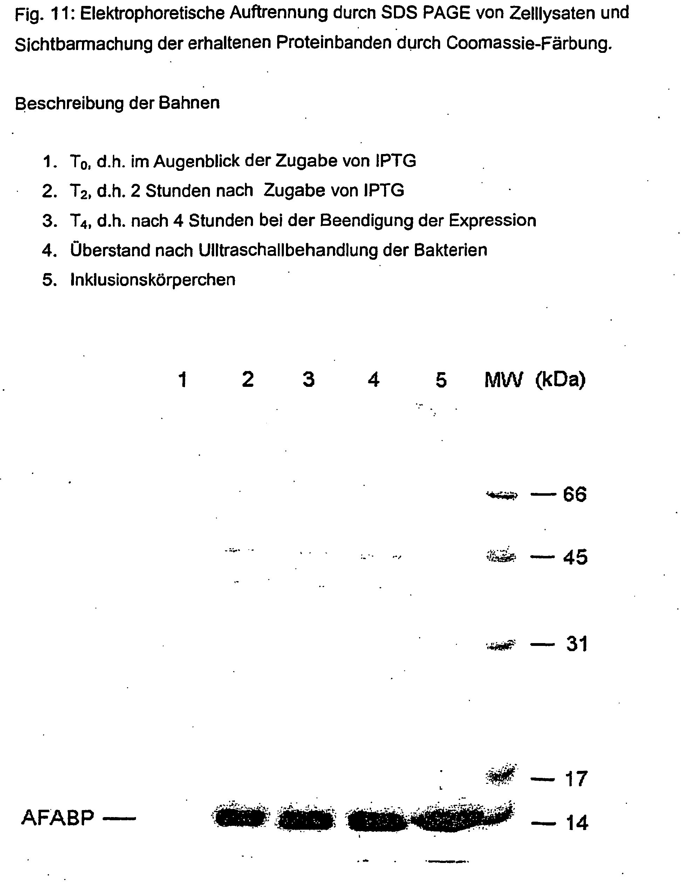

### Expressionsanalyse von A-FABP (Figur 11):

Als Ergebnis der Proteinauftrennung war A-FABP innerhalb der Bahnen 2-5 als deutliche Proteinbande detektierbar, während die Kontrollbahn 1 keine entsprechende Bande zeigte.

### Antikörpergewinnung und Aufarbeitung

Gegen A-FABP wurden polyklonale Antikörper hergestellt und affinitätsgereinigt.

### 1. Immunisierung der Tiere

### 1.1. Ziege

Ziege Nr. 589 wurde nach folgendem Schema immunisiert:

| | | |
|---|---|---|
| 1. Tag | 8x intradermal | 1000ug Antigen + CFA, 1:1 |
| 21. Tag | 2x subkutan | 1000ug Antigen + ICFA, 1:1 |
| 28. Tag | 2x subkuttan | 500ug Antigen + ICFA, 1:1 |
| 35. Tag | 2x subkutan | 500ug Antigen + ICFA, 1:1 |
| 45. Tag | Blutabnahme | 160 ml Blut |
| 105. Tag | 2x subkutan | 500ug Antigen + ICFA, 1:1 |
| 112. Tag | 2x subkutan | 500ug Antigen + ICFA, 1:1 |
| 122. Tag | Blutabnahme | 200 ml Blut |
| 182. Tag | 2x subkutan | 500ug Antigen + ICFA, 1:1 |
| 189. Tag | 2x subkutan | 500ug Antigen + ICFA, 1:1 |
| 199. Tag | Blutabnahme | 200 ml Blut |

| | | |
|---|---|---|
| CFA- Komplettes Freund-Adjuvans IFCA- Inkomplettes Freund-Adjuvans | | |

### 1.2. Kaninchen

Kaninchen Nr. 68 und 69 wurden nach folgendem Schema immunisiert:

| | | |
|---|---|---|
| 1. Tag | 8x intradermal | 200ug Antigen + CFA, 1:1 |
| 21. Tag | 2x subkutan | 200ug Antigen + ICFA, 1:1 |
| 28. Tag | 2x subkuttan | 100ug Antigen + ICFA, 1:1 |
| 35. Tag | 2x subkutan | 100ug Antigen + ICFA, 1:1 |
| 45. Tag | Blutabnahme | 50 ml Blut |
| 105. Tag | 2x subkutan | 100ug Antigen + ICFA, 1:1 |
| 112. Tag | 2x subkutan | 100ug Antigen + ICFA, 1:1 |
| 122. Tag | Blutabnahme | 50 ml Blut |
| 182. Tag | 2x subkutan | 100ug Antigen + ICFA, 1:1 |
| 189. Tag | 2x subkutan | 100ug Antigen + ICFA, 1:1 |
| 199. Tag | Blutabnahme | 50 ml Blut |

| | | |
|---|---|---|
| CFA- Komplettes Freund-Adjuvans IFCA- Inkomplettes Freund-Adjuvans | | |

### 2. Serumgewinnung

Blut (sowohl von Ziege als auch Kaninchen) wurde bei 4° C und bei 2400 G für 20 Minuten zentrifugiert, und das gewonnene Serum bei -20° C gelagert.

### 3. Affinitätsreinigung

### 3.1 Herstellung der Affinitätskolonne

Poros AL (Applied Biosystems), 0,45g, wurde nach Anleitung des Herstellers mit 1mg A-FABP bestückt.

### 3.2. Antikörperaufreinigung

Antikörper wurden im 0,1 M PBS, pH 7,4 an die Kolonne gebunden und mit 0, 1 PBS, 13 mM HCl, 0,15mM NaCl eluiert.

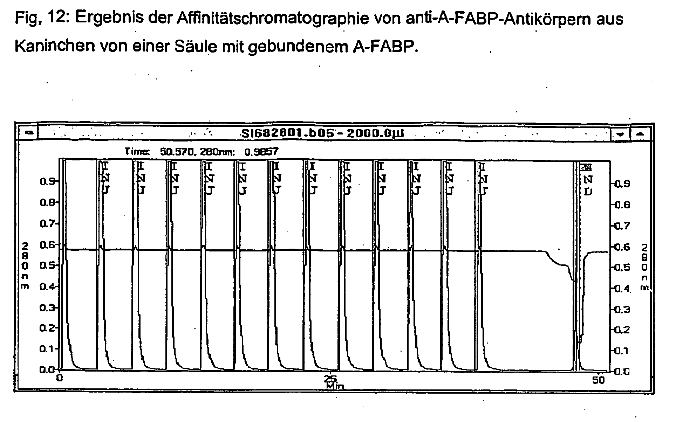

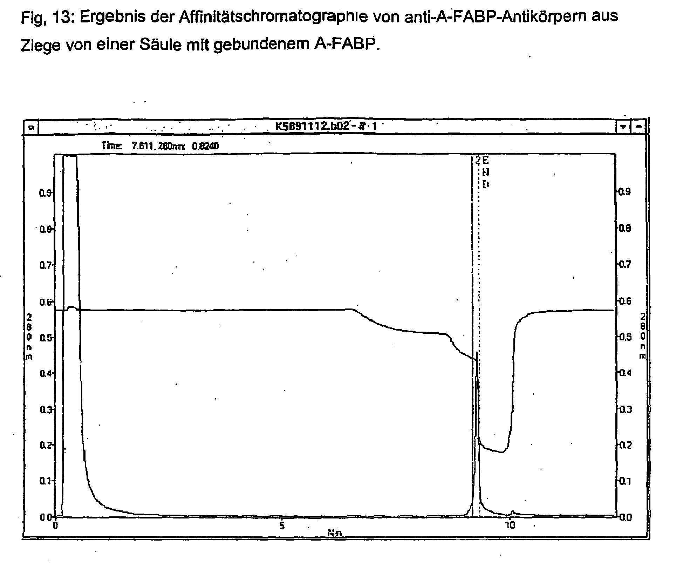

Die Ergebnisse der Affinitätschromatographien sind in Figur 12 (Aufreinigung von Antikörpern aus Kaninchen) und in Figur 13 (Aufreinigung von Antikörpern aus Ziege) dargestellt.

### 3.3. Prüfung der Antikörper auf Reinheit und Titerbestimmung

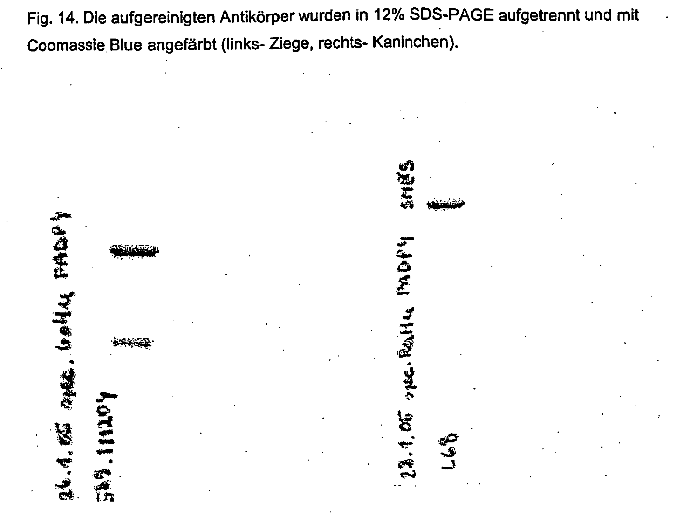

### 3.3.1. Die aufgereinigten Antikörper wurden in 12% SDS-PAGE aufgetrennt und mit Coomassie Blue angefärbt (Figur 14).

### 3.3.2. Titerbestimmung mit indirektem ELISA.

Eine Mikrotiterplatte (Nunc) wurde mit 25ng/Vertiefung A-FABP beschichtet. Affinitätsgereinigte Antikörper wurden in einer Ausgangskonzentration von 1 mg/ml pipettiert und weiter reihenweise 1:3 verdünnt. Titer wurde definiert als eine solche Antikörperverdünnung, die eine Absorbanz von nicht weniger als 1,5 hat:
Ziege:
   Titer: 270 000.-, Menge 4,4 mg
Kaninchen:
   Titer: größer 90 000, Menge: 4,8 mg

### ELISA-Entwicklung:

### EUSA-Platte:

Zur Beschichtung der Mikrotiterplatte wurde der affinitätsgereinigte Ziegenantikörper (4ug/ml) in Hydrogenkarbonatpuffer (0,1 M) verwendet. Nach einer Übernacht-Beschichtung bei 4,0 °C wurden die Platten mit PBS gewaschen und mit 0,5% BSA im TPS, 4% Sacharose 30 Minuten bei Zimmertemperatur blockiert.

### Konjugat:

Affinitätsgereinigter Kaninchenantikörper wurde mit Biotin-LC-LC-NHS-sulfo, Pierce, Cat. No. 21338 nach Anleitung des Herstellers konjugiert und in einer Konzentration von 33 ug/ml eingesetzt.

Streptavidin-HRP Konjugat wurde von der Firma Roche bezogen (Katalognummer 1 089 153)
Kalibrator:
   Rekombinantes humanes A-FABP wurde lyophilisiert und in einer Ausgangskonzentration von 500 ng/ml als Master-Kalibrator verwendet.

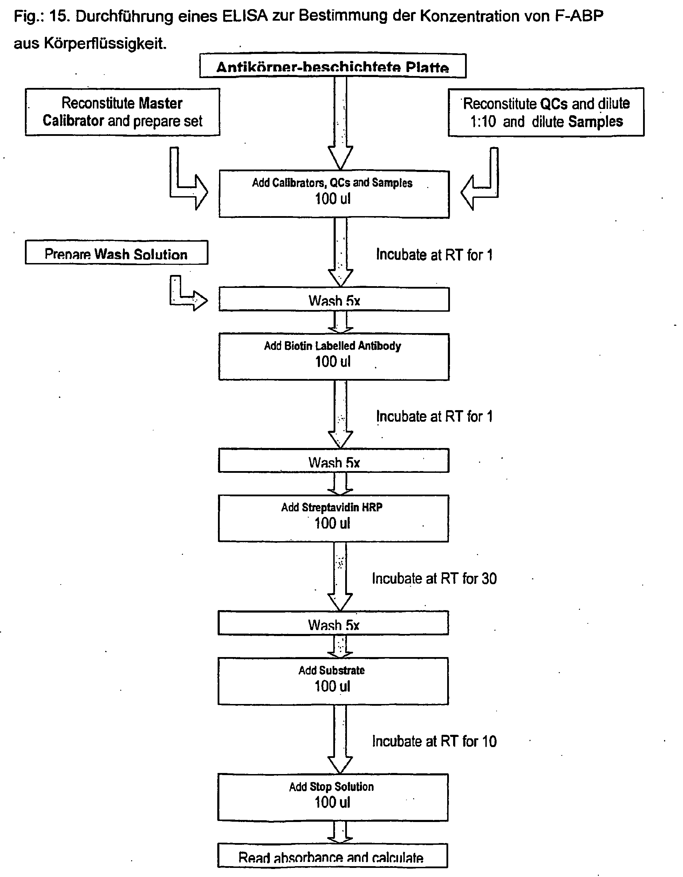

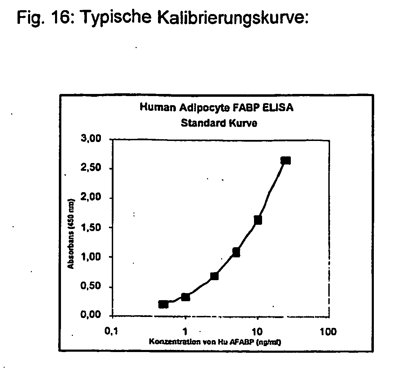

Der eigentliche Elisa-Test wurde, wie in Figur 15 dargestellt, durchgeführt
Abschließend wurden folgende Charakteristika des Tests festgelegt:
a) Typische Kalibrationskurve für humanes A-FABP: Mit dem durchgeführten ELISA ergab sich eine Sensitivität der Konzentrationsmessung von A-FABP von weniger als 100 pg /mL (Figur 16).
b) Recovery (Wiederfindung) / Verdünnung (s. Tabelle 1).

**Tabelle 1**

| Sample | Dilution | Observed (ng/ml) | Expected (ng/ml) | Recovery O/E (%) |
|---|---|---|---|---|
| 1 | - | 36.8 | - | - |
| | 1:2 | 19.6 | 18.4 | 106.5 |
| | 1:4 | 9.9 | 9.2 | 107.6 |
| | 1:8 | 4.9 | 4.6 | 106.5 |
| 2 | - | 28.1 | - | - |
| | 1:2 | 14.1 | 14.1 | 100.0 |
| | 1:4 | 7.8 | 7.0 | 111.0 |
| | 1:8 | 3.9 | 3.5 | 111.0 |

c) Recovery (Wiederfindung) / Zugabe (s. Tabelle 2).

**Tabelle 2**

| Sample | Observed (ng/ml) | Expected (ng/ml) | Recovery O/E (%) |
|---|---|---|---|
| 1 | 8.9 | - | - |
| | 16.5 | 18.9 | 87.3 |
| | 24.1 | 28.9 | 83.4 |
| | 55.5 | 58.9 | 94.2 |
| 2 | 6.8 | - | - |
| | 15.4 | 16.8 | 91.7 |
| | 24.2 | 26.8 | 90.3 |
| | 53.1 | 36.8 | 93.5 |

d) Präzisions-Intraassay (s. Tabelle 3)

**Tabelle 3**

| Sample | Mean (ng/ml) | SD (ng/ml) | CV (%) |
|---|---|---|---|
| 1 | 13.9 | 0.92 | 6.6 |
| 2 | 27.3 | 1.08 | 3.9 |

e) Präzisions-Intraassay (s. Tabelle 4).

**Tabelle 4**

| Sample | Mean (ng/ml) | SD (ng/ml) | CV (%) |
|---|---|---|---|
| 1 | 12.5 | 0.32 | 2.6 |
| 2 | 31.1 | 1.58 | 5.1 |

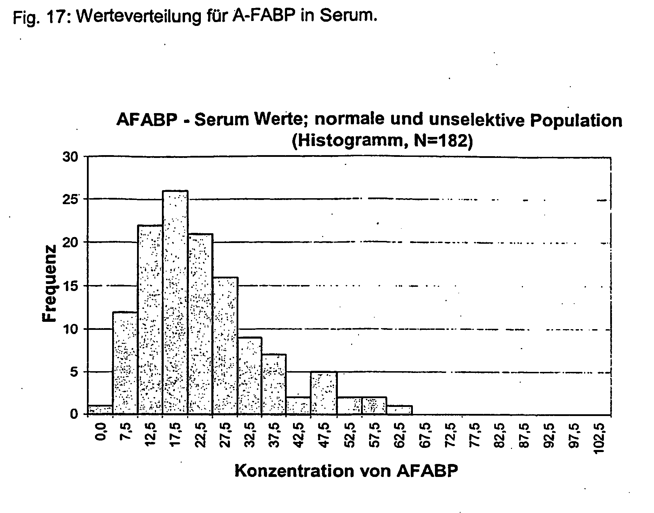

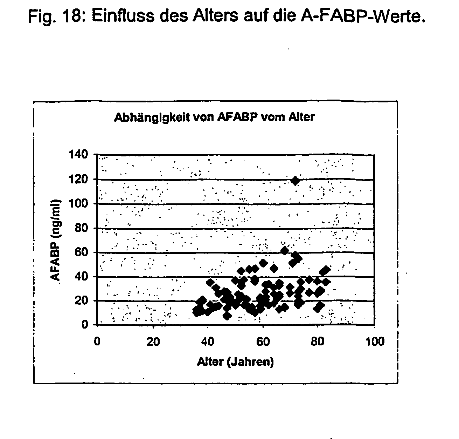

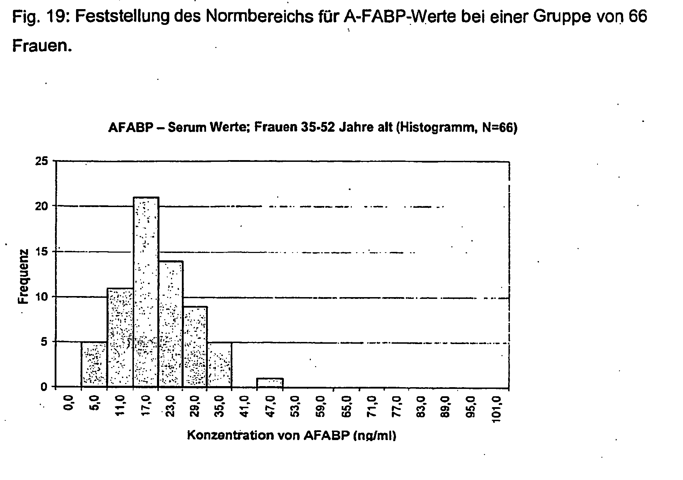

f) Es ergab sich eine Werteverteilung für A-FABP bei gesunden Erwachsenen wie in Figur 17 dargestellt.
g) Der Einfluss des Alters auf die A-FABP-Werte wurde entsprechend Figur 18 festgestellt.
h) Der Einfluss des Geschlechts auf die A-FABP-Werte wurde ermittelt (s. Tabelle 5).

**Tabelle 5**

| | Age | Total cholesterol mmol/l | HDL-chol mmol/l | LDL-chol mmol/l | triglyceride mmol/l | AFABP ng/ml |
|---|---|---|---|---|---|---|
| MEAN (WOMEN): | 45,5 | 5,21 | 1,46 | 3,35 | 1,13 | 21.18 |
| MEAN (MEN): | 43,7 | 5,08 | 1,06 | 3,70 | 1,61 | 21,44 |

i) Bei einer Gruppe von 66 Frauen wurde der Normbereich festgestellt bzw. festgelegt (s. auch Figur 18):
Minimum: 7,7 ng/ml
Maximum: 45,1 ng/ml
Mean: 19, 58 ng/ml
Standard Deviation: 8,16
Normal Range (x+/- 2s): 19,8 +/- 16,32 ng/ml

## Patentansprüche

1. Verfahren zur Diagnose des metabolischen Syndroms,
**dadurch gekennzeichnet, dass** die Konzentration der adipozytären Form des fatty acid binding protein (A-FABP) in Serum oder Plasma bestimmt und als Indikator für die Fettsäuretransportkapazität benutzt wird.

2. Verfahren nach Anspruch 1, **dadurch gekennzeichnet, dass** die Konzentration von A-FABP in Serum bestimmt wird.

3. Verfahren nach einem der Ansprüche 1-2, **dadurch gekennzeichnet, dass** zur Bestimmung der Konzentration des A-FABP ein immunometrisches System verwendet wird.

4. Verfahren nach Anspruch 3, **dadurch gekennzeichnet, dass** Trägermaterial verwendet wird, auf dem Substanzen immobilisiert sind, die Affinität zu A-FABP oder Teilen davon haben.

5. Verfahren nach Anspruch 4, **dadurch gekennzeichnet, dass** die immobilisierten Substanzen mit Affinität zu A-FABP oder Teilen davon als Antikörper gebildet sind.

6. Verfahren nach Anspruch 4, **dadurch gekennzeichnet, dass** das Trägermaterial zumindest teilweise planar gebildet ist.

7. Verfahren nach einem der Ansprüche 4-5, **dadurch gekennzeichnet, dass** das Trägermaterial von einer ELISA-Platte umfaßt wird.

8. Verfahren nach einem der Ansprüche 3-6, **dadurch gekennzeichnet, dass** das immunometrische System ein ELISA ist.

9. Verfahren nach Anspruch 7, **dadurch gekennzeichnet, dass** der ELISA anti-A-FABP-Antikörper, sekundäre Detektionsantikörper, Trägermaterialien, Wasch-, Inkubations- und Detektionslösungen enthält.

10. Verfahren nach einem der Ansprüche 3-9, **dadurch gekennzeichnet, dass** zur Auswertung ein Scanner oder ELISA-Reader verwendet wird.

11. Verfahren nach einem der Ansprüche 1-9, **dadurch gekennzeichnet, dass** die Konzentration von A-FABP mit wenigstens einer weiteren Kenngröße, nämlich dem Alter oder Geschlecht, dem Body-Mass-Index oder Komponenten des Stoffwechsels ausgewählt aus der Gruppe Insulin, Glukose, Triglyceride, QUICKI, Adiponectin, Leptin, Gesamt-Cholesterin, HDL-Cholesterin, LDL-Cholesterin und E-FABP in Bezug gesetzt wird.

12. Verfahren nach einem der Ansprüche 1-11, **dadurch gekennzeichnet, dass** ein Testkit verwendet wird, der ein geeignetes Trägermaterial enthält, auf dem Substanzen mit Affinität zu A-FABP oder Teilen davon immobilisiert sind.

13. Verfahren nach Anspruch 12, **dadurch gekennzeichnet, dass** die immobilisierten Substanzen mit Affinität zu A-FABP oder Teilen davon als Antikörper gebildet sind.

14. Verfahren nach den Ansprüchen 12-13, **dadurch gekennzeichnet, dass** das Trägermaterial zumindest teilweise planar gebildet ist.

15. Verfahren nach den Ansprüchen 12-14, **dadurch gekennzeichnet, dass** das Trägermaterial von einer ELISA-Platte umfaßt wird.

16. Verfahren nach den Ansprüchen 12-15, **dadurch gekennzeichnet, dass** das Testkit Trägermaterial, Substanzen und Lösungen beinhaltet, die zur Durchführung eines ELISA geeignet sind.

17. Verfahren nach Anspruch 16, **dadurch gekennzeichnet, dass** das Testkit anti-A-FABP-Antikörper, sekundäre Detektionsantikörper, Trägermaterialien, Wasch-, Inkubations- und Detektionslösungen enthält.

18. Verfahren nach einem der Ansprüche 16-17, **dadurch gekennzeichnet, dass** das Testkit wenigstens eine Eichlösung beinhaltet, die rekombinantes A-FABP enthält.

19. Verwendung von A-FABP als Serummarker für die Diagnose des metabolischen Syndroms.

## Claims

1. Method for the diagnosis of the metabolic syndrome, **characterised in that** the concentration of the adipocyte form of the fatty acid binding protein (A-FABP) is determined in serum or plasma and is used as an indicator for the fatty acid transport capacity.

2. Method according to claim 1, **characterised in that** the concentration of A-FABP is determined in serum.

3. Method according to one of the claims 1-2, **characterised in that** an immunometric system is used for determining the concentration of A-FABP.

4. Method according to claim 3, **characterised in that** carrier material on which substances with affinity to A-FABP or to parts thereof have been immobilised is used.

5. Method according to claim 4, **characterised in that** the immobilised substances with affinity to A-FABP or to parts thereof are formed as antibodies.

6. Method according to claim 4, **characterised in that** the carrier material is formed at least partially planar.

7. Method according to one of the claims 4-5, **characterised in that** the carrier material is contained in an ELISA plate.

8. Method according to one of the claims 3-6, **characterised in that** the immunometric system is an ELISA.

9. Method according to claim 7, **characterised in that** the ELISA contains anti-A-FABP antibodies, secondary detection antibodies, carrier materials, washing, incubation and detection solutions.

10. Method according to one of the claims 3-9, **characterised in that** a scanner or ELISA reader is used for the analysis.

11. Method according to one of the claims 1-9, **characterised in that** the concentration of A-FABP is placed into reference with at least one further characteristic, namely the age or gender, body mass index or components of the metabolism selected from the group of insulin, glucose, triglycerides, QUICKI, adiponectin, leptin, whole cholesterol, HDL cholesterol, LDL cholesterol, and E-FABP.

12. Method according to one of the claims 1-11, **characterised in that** a test kit containing a suitable carrier material on which substances with affinity to A-FABP or to parts thereof have been immobilised is used.

13. Method according to claim 12, **characterised in that** the immobilised substances with affinity to A-FABP or to parts thereof are formed as antibodies.

14. Method according to claims 12-13, **characterised in that** the carrier material is formed at least partially planar.

15. Method according to claims 12-14, **characterised in that** the carrier material is contained in an ELISA plate.

16. Method according to claims 12-15, **characterised in that** the test kit contains carrier material, substances and solutions suitable for the implementation of an ELISA.

17. Method according to claim 16, **characterised in that** the test kit contains anti-A-FABP antibodies, secondary detection antibodies, carrier materials, washing, incubation and detection solutions.

18. Method according to one of the claims 16-17, **characterised in that** the test kit contains at least one calibration solution containing recombinant A-FABP.

19. Use of A-FABP as a serum marker for the diagnosis of the metabolic syndrome.

## Revendications

1. Procédé de diagnostic du syndrome métabolique, **se caractérisant par le fait que** la concentration de la forme adipocytaire de la protéine fatty acide binding protein (A-FABP) dans le sérum ou le plasma est déterminée et utilisée comme indicateur de la capacité de transport des acides gras.

2. Procédé selon la revendication 1, **se caractérisant par le fait que** la concentration en A-FABP dans le sérum est déterminée.

3. Procédé selon un des revendications 1-2, **se caractérisant par le fait qu'**un système immunométrique est utilisé pour déterminer la concentration en A-FABP.

4. Procédé selon la revendication 3, **se caractérisant par le fait que** l'on utilise un matériau support sur lequel des substances qui ont une affinité avec A-FABP ou des parties de cette protéine, sont immobilisées.

5. Procédé selon la revendication 4, **se caractérisant par le fait que** les substances immobilisées qui ont une affinité avec A-FABP ou des parties de cette protéine, sont constituées sous forme d'anticorps.

6. Procédé selon la revendication 4, **se caractérisant par le fait que** le matériau support est constitué du moins partiellement sous forme planaire.

7. Procédé selon l'une des revendications 4-5, **se caractérisant par le fait que** la matériau support est entouré d'une plaque ELISA.

8. Procédé selon l'une des revendications 3-6, **se caractérisant par le fait que** le système immunométrique est un ELISA.

9. Procédé selon la revendication 7, **se caractérisant par le fait que** l'ELISA contient des anticorps anti-A-FABP, des anticorps de détection secondaires, des matériaux supports, des solutions de lavage, d'incubation et de détection.

10. Procédé selon l'une des revendications 3-9, **se caractérisant par le fait qu'**un scanner ou un lecteur ELISA est utilisé pour l'évaluation.

11. Procédé selon l'une des revendications 1-9, **se caractérisant par le fait que** la concentration en A-FABP est mise en rapport avec au moins une autre valeur de référence, à savoir l'âge ou le sexe, le Body-Mass-Index ou des composants du métabolisme sélectionnés parmi le groupe des insulines, glucoses, triglycérides, QUICKI, adiponectines, leptines, cholestérol global, cholestérol HDL, cholestérol LDL et E-FABP.

12. Procédé selon l'une des revendications 1-11, **se caractérisant par le fait que** l'on utilise un kit de test qui contient un matériau support approprié sur lequel des substances qui ont une affinité avec A-FABP ou des parties de cette protéine, sont immobilisées.

13. Procédé selon la revendication 12, **se caractérisant par le fait que** les substances immobilisées qui ont une affinité avec A-FABP ou des parties de cette protéine, sont constituées sous forme d'anticorps.

14. Procédé selon les revendications 12-13, **se caractérisant par le fait que** le matériau support est constitué du moins partiellement sous forme planaire.

15. Procédé selon l'une des revendications 12-14, **se caractérisant par le fait que** le matériau support est entouré d'une plaque ELISA.

16. Procédé selon les revendications 12-15, **se caractérisant par le fait que** le kit de test contient le matériau support, les substances et solutions qui sont appropriées à l'exécution d'un ELISA.

17. Procédé selon la revendication 16, **se caractérisant par le fait que** le kit de test contient des anticorps anti-A-FABP, des anticorps de détection secondaires, des matériaux supports, des solutions de lavage, d'incubation et de détection.

18. Procédé selon l'une des revendications 16-17, **se caractérisant par le fait que** le kit de test contient au moins une solution étalon qui contient la protéine recombinante A-FABP.

19. Emploi de la protéine A-FABP en tant que marqueur de sérum pour le diagnostic du syndrome métabolique.
